# FASCICULE DE BREVET EUROPEEN

(11) **EP 3 231 399 B1**
(45) Date de publication et mention de la délivrance du brevet: **08.05.2019**
(21) Numéro de dépôt: 17165584.8
(22) Date de dépôt: 07.04.2017
(51) Int. Cl.: A61F 2/64, A61F 2/66, A61F 2/68, A61F 2/70, A61F 2/76

(54) **GENOU PROTHETIQUE POUR UN PATIENT AMPUTE FEMORAL**
KNIEPROTHESE FÜR EINEN OBERSCHENKELAMPUTIERTEN PATIENTEN
PROSTHETIC KNEE FOR A FEMORAL AMPUTEE PATIENT

(30) Priorité: 08.04.2016 FR 1653120
(43) Date de publication de la demande: 18.10.2017
(73) Titulaire: Commissariat à l'Energie Atomique et aux Energies Alternatives, 75015 Paris (FR); Chabloz Composants, 38170 Seyssinet-Pariset (FR)
(72) Inventeur: Pelisson, Roland, 38660 La Terrasse (FR); Chabloz, Pierre, 38450 Saint Georges de Commiers (FR)
(74) Mandataire: Talbot, Alexandre

(56) Documents cités:
- WO-A1-2008/080232
- FR-A1- 2 464 702
- US-A- 3 599 245

## Description

### Domaine technique

L'invention se rapporte au domaine technique des genoux prothétiques pour un patient amputé fémoral. L'invention trouve notamment son application en gériatrie.

### Etat de la technique antérieure

Un genou prothétique connu de l'état de la technique, du type genou à verrou, comporte :
- une articulation mobile en rotation autour d'un axe perpendiculaire à un plan sagittal du patient entre une position d'extension et une position de flexion du genou prothétique ;
- un mécanisme de verrouillage de l'articulation en position d'extension, lorsque le patient passe d'une position assise à la station debout.

Le mécanisme de verrouillage est classiquement commandé par un câble permettant de déverrouiller l'articulation, c'est-à-dire libérer l'articulation de la position d'extension, lorsque le patient souhaite passer de la station debout à une position assise.

Un tel genou prothétique à verrou n'est pas entièrement satisfaisant. En effet, un inconvénient majeur est le déverrouillage brusque de l'articulation lorsque le patient souhaite s'asseoir. Un patient âgé, peu alerte, est alors susceptible de perdre l'équilibre. Le patient âgé appréhende cette situation et est donc peu enclin à utiliser régulièrement ce genou prothétique.

Ainsi, l'invention vise à proposer un genou prothétique sécurisant pour un patient âgé, et peu coûteux afin d'être pris en charge par un organisme de santé tel que la caisse primaire d'assurance maladie en France.

Le document US 3599245 décrit un mécanisme de genou qui comporte un axe de genou permettant de définir la flexion du tibia par rapport à la cuisse. Une bande de frottement est installée autour d'un tambour rotatif. La tension au sein de la bande de frottement est réglée par une vis qui passe au travers de la bande de frottement.

Le document FR 2464702 décrit un genou prothétique doté d'un mécanisme de retenue pour s'opposer à la flexion du genou. Le genou prothétique comporte un tambour tournant placé suivant l'axe de rotation du genou, et un ruban flexible de frein enroulé autour du tambour.

### Exposé de l'invention

A cet effet, l'invention a pour objet un genou prothétique pour un patient amputé fémoral, comportant :
- une partie proximale destinée à être reliée à une emboîture prothétique, la partie proximale étant mobile en rotation autour d'un premier axe perpendiculaire à un plan sagittal du patient entre une position d'extension et une position de flexion du genou prothétique ; la partie proximale étant mobile dans un premier sens de rotation de la position d'extension vers la position de flexion, et mobile dans un second sens de rotation opposé de la position de flexion vers la position d'extension ;
- au moins une pièce, la pièce étant fixe lorsque la partie proximale est mue dans le premier sens de rotation ;
- un élément de liaison, agencé pour relier la partie proximale et la pièce, l'élément de liaison présentant une surface de contact avec la pièce ; l'élément de liaison et la pièce étant agencés de sorte que,
   - lorsque la partie proximale est mue dans le premier sens de rotation, l'élément de liaison glisse sur la surface de contact en subissant une première force de frottement,
   - lorsque la partie proximale est mue dans le second sens de rotation, l'élément de liaison glisse sur la surface de contact en subissant une seconde force de frottement strictement inférieure à la première force de frottement.

### Définitions

Par « proximale », on entend la partie qui est située le plus près du membre résiduel du patient amputé fémoral.

Par « distale », on entend la partie qui est la plus éloignée du membre résiduel.

Par « plan sagittal », on entend un plan parallèle au plan sagittal médian du patient.

Lorsqu'un patient âgé souhaite passer de la station debout à une position assise, le genou prothétique selon l'invention opère une flexion se traduisant par le mouvement en rotation de la partie proximale dans le premier sens de rotation. Le glissement avec frottement de l'élément de liaison permet de ralentir le passage de la station debout à la position assise. Ce ralentissement de la flexion du genou prothétique permet de sécuriser le patient âgé qui peut conserver aisément son équilibre jusqu'à l'obtention de la position assise. On peut parler d'un « effet de frein » à la flexion. Ce ralentissement de la flexion du genou prothétique est également bénéfique lorsque le patient souhaite passer d'une position assise à la station debout en s'arrêtant dans au moins une position intermédiaire, afin de s'aider par exemple d'abord d'accoudoirs, puis d'une canne ou de béquilles. En effet, dans la ou les positions intermédiaires, une reprise de poids sur le genou prothétique s'accompagne alors de l' « effet de frein » qui sécurise le patient.

Le fait que la seconde force de frottement soit strictement inférieure à la première force de frottement permet part ailleurs de faciliter le passage d'une position assise vers la station debout par rapport au passage de la station debout vers une position assise. Cela encourage donc le patient âgé à se lever et à se déplacer.

Par ailleurs, le genou prothétique selon l'invention est peu coûteux car le ralentissement de la flexion est obtenu par simple friction, ne nécessitant aucun système complexe tel qu'un vérin hydraulique.

Le genou prothétique selon l'invention peut comporter une ou plusieurs des caractéristiques suivantes.

Selon une caractéristique de l'invention, les première et seconde forces de frottement, notées respectivement f₁ et f₂, présentent un ratio f₁/f₂ supérieur ou égal à 10.
Ainsi, un avantage procuré par tel ratio est d'effectuer le plus facilement possible le passage d'une position assise vers la station debout en offrant le moins de résistance possible pour le patient, et ce tout en conservant un ralentissement satisfaisant de la flexion du genou prothétique de la station debout vers une position assise, ou d'une position assise vers une position intermédiaire avec une reprise de poids sur le genou prothétique.

Selon une caractéristique de l'invention, la pièce est mobile lorsque la partie proximale est mue dans le second sens de rotation.
Ainsi, un avantage procuré est de s'affranchir de la résistance de la pièce lors du passage d'une position assise vers la station debout. Il est donc possible d'obtenir un ratio f₁/f₂ très supérieur à 10, par exemple de l'ordre de 20.

Selon une caractéristique de l'invention, le genou prothétique comporte des moyens de mise en tension agencés pour exercer une tension mécanique sur l'élément de liaison.
Ainsi, un avantage procuré est la possibilité de régler la première force de frottement, et par là-même le ralentissement de la flexion du genou prothétique, lorsque l'élément de liaison est souple. Une augmentation (respectivement une baisse) de la tension mécanique exercée sur l'élément de liaison permet de générer une résistance à la flexion plus forte (respectivement moins forte).

Selon une caractéristique de l'invention, le genou prothétique comporte des moyens de commande configurés pour commander les moyens de mise en tension.
Ainsi, un avantage procuré est la possibilité d'envisager de moduler la tension mécanique exercée sur l'élément de liaison durant la flexion du genou prothétique.

Selon une caractéristique de l'invention, les moyens de mise en tension comportent un tendeur rotatif, et les moyens de commande comportent un servomoteur configuré pour modifier un couple exercé sur le tendeur rotatif.
Ainsi, un avantage procuré par le tendeur rotatif est de limiter l'usure de l'élément de liaison (par exemple par rapport à un cavalier entourant l'élément de liaison pour le pincer), en particulier lorsque l'élément de liaison est une cordelette.

Selon une caractéristique de l'invention, le genou prothétique comporte une jauge de contraintes agencée pour mesurer le couple exercé sur le tendeur rotatif.
Ainsi, un avantage procuré est de connaître le couple afin de le piloter par les moyens de commande.

Selon une caractéristique de l'invention, la partie proximale présente un angle de pivotement entre la position d'extension et la position de flexion dans un plan sagittal du patient; le genou prothétique comportant des moyens de mesure agencés pour mesurer l'angle de pivotement, les moyens de mesure étant de préférence sélectionnés dans le groupe comportant un accéléromètre, un gyromètre, un capteur potentiométrique.
Ainsi, un avantage procuré est la possibilité de moduler la tension mécanique exercée sur l'élément de liaison durant la flexion du genou prothétique en fonction de l'angle de pivotement.

Selon une caractéristique de l'invention, le genou prothétique comporte un capteur de force agencé pour mesurer la force exercée par l'élément de liaison sur la pièce.

Selon une caractéristique de l'invention, le genou prothétique comporte des moyens d'avertissement configurés pour avertir le patient dès lors que la force mesurée par le capteur de force dépasse un seuil, les moyens d'avertissement comportant de préférence un vibreur.
Ainsi, un avantage procuré est de donner la possibilité au patient d'anticiper le début de la phase de flexion du genou prothétique.

Selon une caractéristique de l'invention, le genou prothétique comporte des moyens de rappel élastique agencés pour rappeler la partie proximale dans la position d'extension.
Ainsi, un avantage procuré est de faciliter le passage d'une position assise vers la station debout.

Selon une caractéristique de l'invention, l'élément de liaison est sélectionné dans le groupe comportant une sangle, une cordelette, une courroie.
Ainsi, les avantages procurés sont la simplicité et le coût réduit de tels éléments de liaison.

Selon une caractéristique de l'invention, l'élément de liaison comporte une partie enroulée autour de la pièce, la partie enroulée étant de préférence une cordelette. Ainsi, un avantage procuré par la cordelette est de réduire l'encombrement de manière significative, par exemple relativement à une sangle, en l'enroulant autour de la pièce sur une pluralité de tours.

Selon une caractéristique de l'invention, le genou prothétique comporte un ensemble de pièces, les pièces de l'ensemble étant fixes lorsque la partie proximale est mue dans le premier sens de rotation ; l'élément de liaison étant agencé pour relier la partie proximale et l'ensemble de pièces, l'élément de liaison présentant des surfaces de contact avec l'ensemble de pièces ; l'élément de liaison et l'ensemble de pièces étant agencés de sorte que, lorsque la partie proximale est mue dans le premier sens de rotation, l'élément de liaison glisse sur chaque surface de contact en subissant la première force de frottement correspondante.
Ainsi, un avantage procuré est d'augmenter la force de frottement totale subie par l'élément de liaison en augmentant le nombre de pièces fixes lorsque la partie proximale est mue dans le premier sens de rotation, ce qui permet de ralentir d'autant plus la flexion du genou prothétique.

Selon une caractéristique de l'invention, l'élément de liaison et l'ensemble de pièces sont agencés de sorte que l'élément de liaison forme des lacets autour de l'ensemble de pièces.
Ainsi, un avantage procuré par cette configuration géométrique en zigzag (c'est-à-dire que les lacets décrivent une ligne sinueuse présentant des courbes dirigées alternativement en sens contraire) de l'élément de liaison relativement à l'ensemble de pièces est d'augmenter la force de frottement totale subie par l'élément de liaison, tout en conservant une compacité satisfaisante du genou prothétique.

Selon une caractéristique de l'invention, le genou prothétique comporte :
- une partie distale, destinée à être reliée à un pied prothétique, la partie distale étant mobile en rotation autour d'un second axe perpendiculaire à un plan sagittal du patient entre une position d'avancée et une position de recul du pied prothétique ; la partie distale étant mobile dans un premier sens de rotation de la position d'avancée vers la position de recul ;
- un élément de liaison additionnel agencé pour relier la partie proximale et la partie distale de sorte que, lorsque la partie proximale est mue dans le premier sens de rotation, la partie distale est mue dans le premier sens de rotation.
Ainsi, un avantage procuré est de déplacer le genou prothétique vers l'avant du patient lors du passage de la station debout à une position assise. Ce déplacement vers l'avant du genou prothétique pendant la flexion améliore sensiblement la stabilité du patient qui n'a plus à incliner son torse vers l'avant. En effet, en condition d'utilisation, le pied prothétique est fixe, maintenu au sol par le poids du patient. Par conséquent, le mouvement en rotation de la partie distale dans le premier sens de rotation se traduit par un déplacement vers l'avant de la partie proximale, et par la-même du genou prothétique.

Selon une caractéristique de l'invention, l'élément de liaison additionnel et l'ensemble de pièces sont agencés de sorte que l'élément de liaison additionnel forme des lacets autour de l'ensemble de pièces.
Ainsi, un avantage procuré est l'obtention d'une sécurité supplémentaire pendant la marche du patient. Lors du posé du talon du pied prothétique sur un sol, la position d'extension est maintenue en outre par des frottements de l'élément additionnel qui se tend en contact autour de l'ensemble de pièces. En d'autres termes, pendant la marche, le posé du talon tend l'élément de liaison additionnel qui, du fait des lacets, subit un frottement amplifié qui bloque le genou prothétique en position d'extension, ce qui est particulièrement avantageux en l'absence d'électronique de commande. En revanche, à l'arrêt et en appui sur la pointe du pied prothétique, l'élément de liaison additionnel ne subit pas de force de frottement significative.

Seul l'élément de liaison subit une force de frottement permettant d'obtenir le ralentissement de la flexion du genou prothétique.

Selon une caractéristique de l'invention, l'élément de liaison additionnel est sélectionné dans le groupe comportant une sangle, une cordelette, une courroie. Ainsi, les avantages procurés sont la simplicité et le coût réduit de tels éléments de liaison additionnels.

Selon une caractéristique de l'invention, la partie proximale et la partie distale présentent des vitesses de rotation dans le premier sens de rotation notées respectivement Vp₁ et Vd₁ ; le genou prothétique comportant des organes adaptés pour recevoir l'élément de liaison additionnel, les organes étant configurés de sorte que Vd₁ est strictement inférieure à Vp₁ avec de préférence *Vp*₁/*Vd*₁ ≥ 3.
Ainsi, un avantage procuré est d'obtenir, par démultiplication, un déplacement important de la partie proximale (et par là-même du genou prothétique) vers l'avant du patient lors du passage de la station debout à une position assise, et ce tout en conservant une compacité satisfaisante de la partie distale.

Selon une caractéristique de l'invention, la partie proximale présente une forme oblongue dans un plan sagittal du patient.
Ainsi, un avantage procuré par la forme oblongue (c'est-à-dire plus longue que large) est d'obtenir un déplacement de la partie proximale vers l'avant du patient avec :
- une amplitude importante dès le début de la flexion du genou prothétique grâce à l'élément de liaison additionnel se déplaçant en contact avec la portion large de la partie proximale, de dimension restreinte ;
- une amplitude plus faible jusqu'à la fin de la flexion grâce à l'élément de liaison additionnel se déplaçant en contact avec la portion longue de la partie proximale, de dimension plus importante.
Cet avantage est obtenu indépendamment de l'élément de liaison permettant l' « effet de frein ». Une telle forme oblongue est susceptible de conférer l'avantage précité quel que soit le mécanisme permettant l' « effet de frein ».

Cela étant, un avantage supplémentaire procuré est qu'en fin de flexion, l'élément de liaison peut être fortement tendu, ce qui conduit à durcir l' « effet de frein ».

### Brève description des dessins

D'autres caractéristiques et avantages apparaîtront dans l'exposé détaillé de différents modes de réalisation de l'invention, l'exposé étant assorti d'exemples et de référence aux dessins joints.

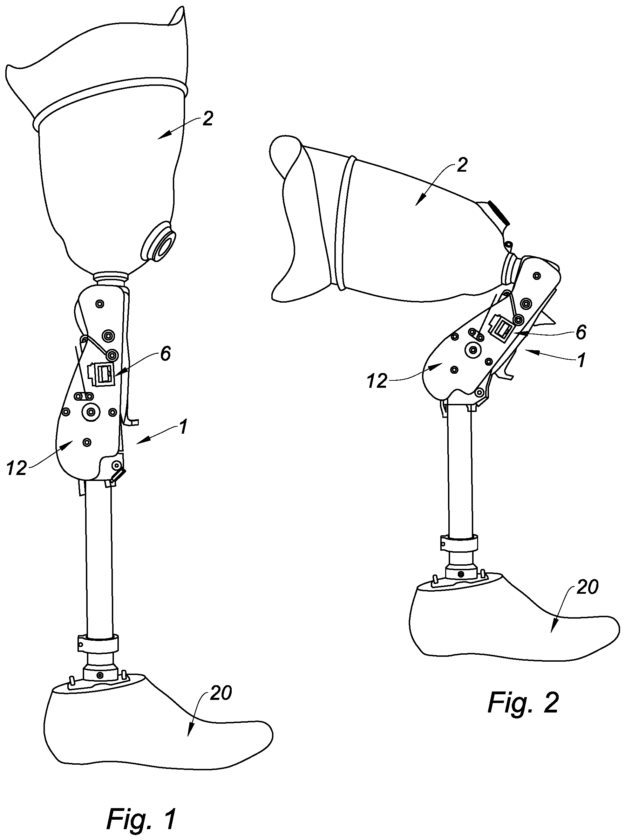

Figure 1 est une vue de côté d'un genou prothétique selon l'invention, sur lequel sont montés une emboîture prothétique et un pied prothétique, le genou prothétique étant dans une position d'extension.
Figure 2 est une vue analogue à la figure 1 où le genou prothétique est dans une position de flexion.
Figure 3 est une vue de côté d'un genou prothétique en position d'extension, selon un premier mode de réalisation de l'invention. Un flanc est démonté pour une meilleure compréhension.
Figure 4 est une vue analogue à la figure 3, où le genou prothétique est dans une position de flexion.
Figure 5 est une vue schématique en perspective d'un genou prothétique en position d'extension, selon le premier mode de réalisation de l'invention.
Figure 6 est une vue analogue à la figure 5, où le genou prothétique est dans une position de flexion.
Figure 7 est une vue schématique de côté d'un genou prothétique selon un deuxième mode de réalisation.
Figure 8 est une vue schématique en coupe sagittale d'un genou prothétique selon un troisième mode de réalisation.
Figure 9a est une vue de côté à l'échelle agrandie d'un genou prothétique selon l'invention illustrant un mode de réalisation de la commande des moyens de mise en tension.
Figure 9b est une vue analogue à la figure 9a illustrant un autre mode de réalisation de la commande.
Figure 10 est une vue schématique d'un capteur de force à jauges résistives équipant un genou prothétique selon l'invention.
Figure 11 est une vue schématique d'un genou prothétique selon l'invention (en l'absence d'élément de liaison), équipé d'une carte électronique.
Figure 12 est une vue partielle d'un genou prothétique selon l'invention, illustrant des moyens de rappel élastique pour rappeler la partie proximale dans la position d'extension.
Figure 13 est une vue partielle d'un genou prothétique selon l'invention, illustrant des moyens de mesure d'un angle de pivotement entre la position d'extension et la position de flexion dans un plan sagittal du patient.

Les éléments identiques ou assurant la même fonction porteront les mêmes références pour les différents modes de réalisation, par souci de simplification.

### Exposé détaillé des modes de réalisation

L'objet de l'invention est un genou prothétique 1 pour un patient amputé fémoral, comportant :
- une partie proximale 10 destinée à être reliée à une emboîture prothétique 2, la partie proximale 10 étant mobile en rotation autour d'un premier axe X'-X perpendiculaire à un plan sagittal du patient entre une position d'extension et une position de flexion du genou prothétique ; la partie proximale 10 étant mobile dans un premier sens de rotation de la position d'extension vers la position de flexion, et mobile dans un second sens de rotation opposé de la position de flexion vers la position d'extension ;
- au moins une pièce 3, la pièce 3 étant fixe lorsque la partie proximale 10 est mue dans le premier sens de rotation ;
- un élément de liaison 4, agencé pour relier la partie proximale 10 et la pièce 3, l'élément de liaison 4 présentant une surface de contact 40 avec la pièce 3 ; l'élément de liaison 4 et la pièce 3 étant agencés de sorte que, lorsque la partie proximale 10 est mue dans le premier sens de rotation, l'élément de liaison 4 glisse sur la surface de contact 40 en subissant une première force de frottement.

### Structure du genou prothétique

Le genou prothétique 1 comporte préférentiellement deux bords latéraux 12 entre lesquels est montée la partie proximale 10. Les bords latéraux 12 peuvent être réalisés sous la forme de plaques. Les bords latéraux 12 peuvent être des flancs métalliques, par exemple en aluminium. Les bords latéraux 12 peuvent être également réalisés en carbone. Les bords latéraux 12 s'étendent préférentiellement dans un plan sagittal du patient.

### Elément de liaison

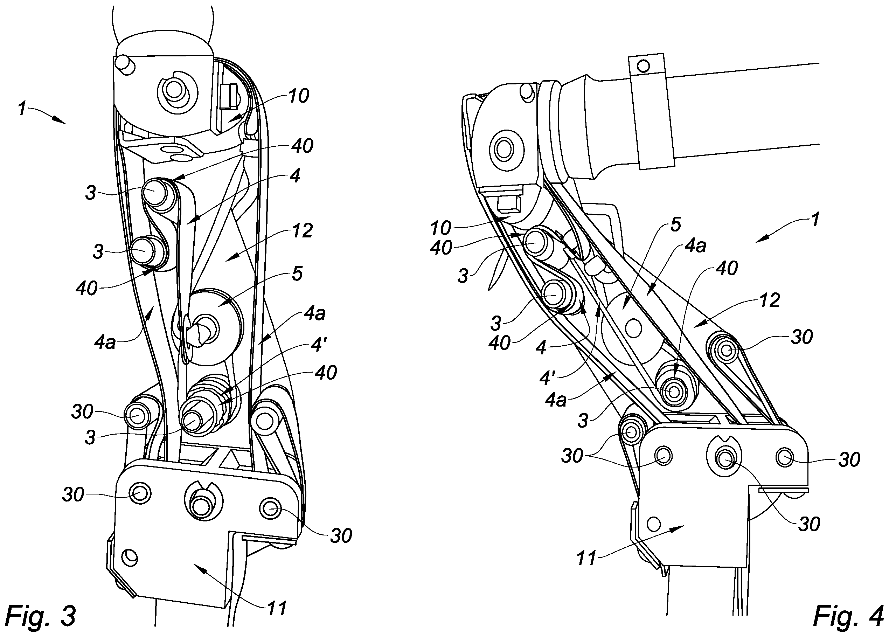

L'élément de liaison 4 est préférentiellement souple. L'élément de liaison 4 est avantageusement sélectionné dans le groupe comportant une sangle, une cordelette, une courroie. L'élément de liaison 4 comporte avantageusement une partie enroulée autour de la pièce 3. La partie de l'élément de liaison 4 enroulée autour de la pièce 3 est de préférence une cordelette 4'. Comme illustré aux figures 3 à 6, l'élément de liaison 4 peut être une sangle prolongée par la cordelette 4'. La cordelette 4' permet de réduire l'encombrement de manière significative par rapport à une sangle. L'élément de liaison 4 est avantageusement résistant à la traction et à l'usure. Lorsque l'élément de liaison 4 est une sangle, celle-ci est par exemple réalisée en Cordura®. L'élément de liaison 4 peut être guidé à l'aide d'une plaque de guidage.

### La (ou les) pièce(s) fixe(s) lorsque la partie proximale est mue dans le premier sens de rotation

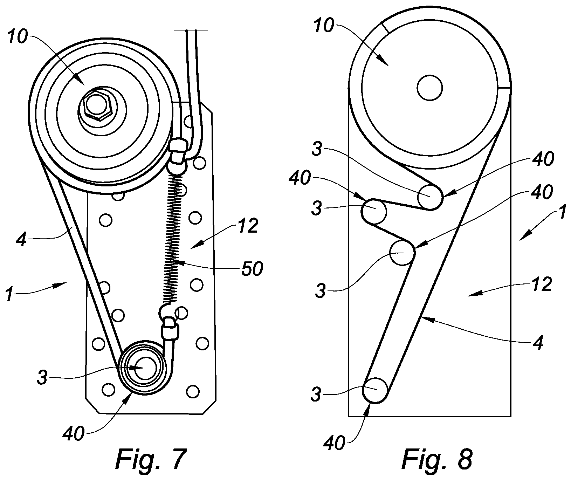

Comme illustré à la figure 7, le genou prothétique 1 peut comporter une unique pièce 3, l'unique pièce 3 étant fixe lorsque la partie proximale 10 est mue dans le premier sens de rotation. Plus précisément, l'unique pièce 3 peut être fixée à un bord latéral 12 du genou prothétique 1. L'élément de liaison 4 peut être une cordelette 4' enroulée autour de la partie proximale 10 et autour de l'unique pièce 3, de préférence sur plusieurs tours. L'unique pièce 3 est de préférence de forme tubulaire. L'unique pièce 3 peut être réalisée dans un métal ou un alliage métallique tel que l'acier.

Comme illustré aux figures 3 à 6, 8 et 11-12, le genou prothétique 1 peut comporter un ensemble de pièces 3, les pièces 3 de l'ensemble étant fixes lorsque la partie proximale 10 est mue dans le premier sens de rotation. Plus précisément, les pièces 3 de l'ensemble peuvent comporter chacune une portion fixe lorsque la partie proximale 10 est mue dans le premier sens de rotation. La portion fixe est préférentiellement fixée à un bord latéral 12 du genou prothétique 1. L'élément de liaison 4 est agencé pour relier la partie proximale 10 et l'ensemble de pièces 3. L'élément de liaison 4 présente des surfaces de contact 40 avec l'ensemble de pièces 3. Chaque pièce 3 de l'ensemble est préférentiellement de forme tubulaire. Plus précisément, la portion fixe des pièces 3 de l'ensemble est préférentiellement de forme tubulaire. Chaque pièce 3 de l'ensemble peut être réalisée dans un métal ou un alliage métallique tel que l'acier.

### Coopération entre l'élément de liaison et la ou les pièces

Comme illustré à la figure 7, dans le cas d'une unique pièce 3, L'élément de liaison 4 et la pièce 3 sont avantageusement agencés de sorte que, lorsque la partie proximale 10 est mue dans le second sens de rotation, l'élément de liaison 4 glisse sur la surface de contact 40 en subissant une seconde force de frottement strictement inférieure à la première force de frottement. Les première et seconde forces de frottement, notées respectivement f₁ et f₂, présentent avantageusement un ratio f₁/f₂ supérieur ou égal à 10. Pour ce faire, à titre d'exemple, un ressort 50 est agencé pour relier deux extrémités de l'élément de liaison 4. La seconde force de frottement f₂ est strictement inférieure à la première force de frottement f₁ car le ressort 50 est agencé pour exercer une tension uniquement dans le premier sens de rotation. Le ratio f₁/f₂ dépend de la raideur du ressort 50 qui conditionne la tension, et par là-même le ralentissement de la flexion du genou prothétique 1.
Le ressort 50 peut être remplacé par un amortisseur dont le rôle est de prévenir une flexion trop rapide du genou prothétique 1. Le ressort 50 peut également être associé à un tel amortisseur.

Selon une alternative, l'unique pièce 3 est avantageusement mobile lorsque la partie proximale 10 est mue dans le second sens de rotation. Le ratio f₁/f₂ devient alors très supérieur à 10. La seconde force de frottement f₂ peut alors être quasiment nulle si l'unique pièce 3 fonctionne de façon analogue à une roue libre.

Comme illustré aux figures 3 à 6, 8, et 11-12, dans le cas d'un ensemble de pièces 3, l'élément de liaison 4 et l'ensemble de pièces 3 sont agencés de sorte que, lorsque la partie proximale 10 est mue dans le premier sens de rotation, l'élément de liaison 4 glisse sur chaque surface de contact 40 en subissant la première force de frottement correspondante. L'élément de liaison 4 et l'ensemble de pièces 3 sont avantageusement agencés de sorte que l'élément de liaison 4 forme des lacets autour de l'ensemble de pièces 3. Au moins une pièce 3 de l'ensemble présente avantageusement une portion mobile lorsque la partie proximale 10 est mue dans le second sens de rotation. Pour ce faire, un roulement 31 de roue libre, formant la portion mobile, peut être monté sur la portion fixe de la pièce 3. Un tel roulement 31 de roue libre permet de réduire significativement les forces de frottement subies par l'élément de liaison 4 lorsque la partie proximale 10 est mue dans le second sens de rotation. Dans les modes de réalisation où l'élément de liaison 4 comporte une cordelette 4', la cordelette 4' est avantageusement enroulée autour de la portion mobile de la pièce 3.

Selon un mode de réalisation particulier, la vitesse de rotation d'au moins une des pièces 3 peut être commandée par un moteur (non représenté), de sorte à aider le patient à se lever lorsqu'il le souhaite. Le moteur peut être actionné directement par le patient, ou actionné par un capteur de force dès que la force mesurée dépasse un seuil traduisant le souhait du patient de passer en position debout.

### Rappel en position d'extension

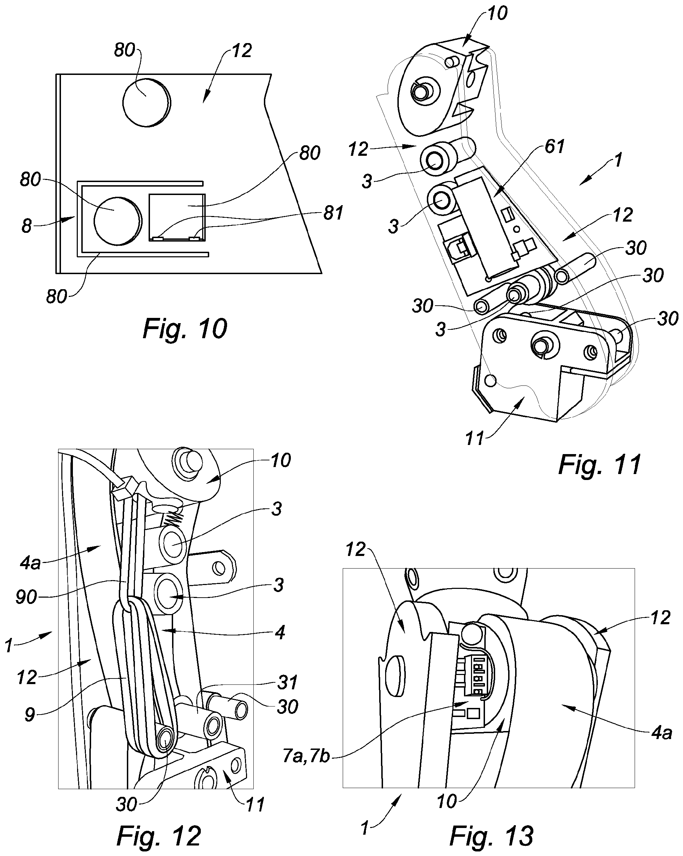

Le genou prothétique 1 comporte avantageusement des moyens de rappel élastique agencés pour rappeler la partie proximale 10 dans la position d'extension. A titre d'exemple illustré à la figure 12, les moyens de rappel élastique comportent un élastique 9 monté sur une bride 90, la bride 90 étant fixée à la partie proximale 10. A titre de variante, les moyens de rappel élastique peuvent comporter un câble et un ressort. Les moyens de rappel élastique sont facultatifs, en particulier lorsque l'on utilise un tendeur rotatif 5 et un roulement 31 de roue libre monté sur une pièce 3, le rappel dans la position d'extension pouvant s'effectuer par simple gravité du fait du ratio f₁/f₂ supérieur ou égal à 10, voire très supérieur à 10.

### Mise en tension de l'élément de liaison et modulation de la tension mécanique

Le genou prothétique 1 comporte avantageusement des moyens de mise en tension agencés pour exercer une tension mécanique sur l'élément de liaison 4. Dans les modes de réalisation où l'élément de liaison 4 comporte une cordelette 4', les moyens de mise en tension sont avantageusement agencés pour exercer une tension mécanique sur la cordelette 4'.
Comme illustré à la figure 7 et évoqué précédemment, les moyens de mise en tension peuvent comporter un ressort 50 reliant deux extrémités de l'élément de liaison 4.
Comme illustré aux figures 3 à 6, les moyens de mise en tension comportent avantageusement un tendeur rotatif 5. Selon une alternative, les moyens de tension peuvent comporter un cavalier entourant l'élément de liaison 4 de manière à le pincer.

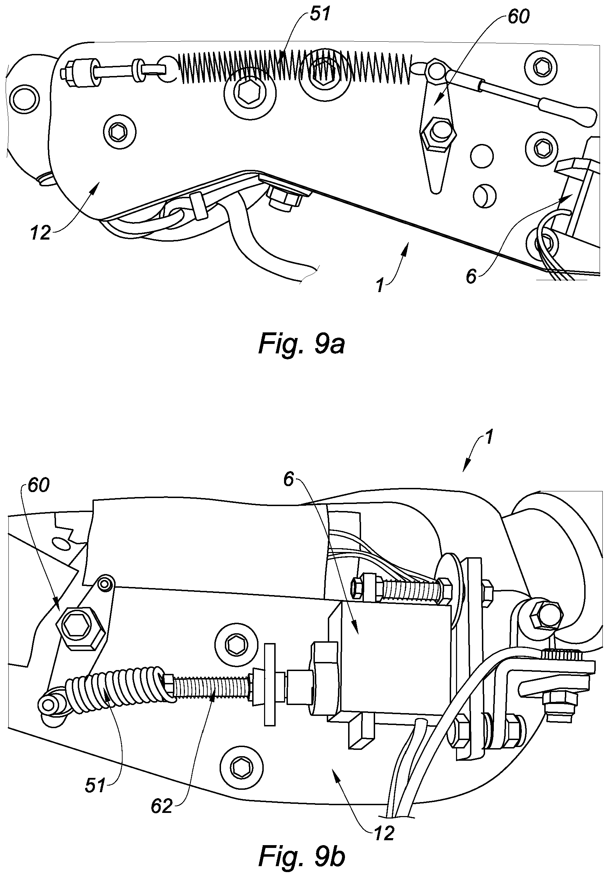

Le genou prothétique 1 comporte avantageusement des moyens de commande configurés pour commander les moyens de mise en tension. Les moyens de commande peuvent être agencés sur une surface d'un bord latéral 12 du genou prothétique 1. Comme illustré aux figures 9a et 9b, les moyens de commande comportent avantageusement un servomoteur 6 configuré pour modifier un couple exercé sur le tendeur rotatif 5, c'est-à-dire augmenter ou réduire le couple. Pour ce faire, le servomoteur 6 est configuré pour actionner un palonnier 60 exerçant un couple sur le tendeur rotatif 5. Le servomoteur 6 peut actionner le palonnier 60 via une biellette élastique. Selon une variante illustrée à la figure 9b, le servomoteur 6 peut actionner le palonnier 60 en modifiant la tension d'un ressort 51. Pour ce faire, le servomoteur 6 peut comporter une vis sans fin 62 montée à une extrémité du ressort 51. L'autre extrémité du ressort 51 est montée sur le palonnier 60. La vis sans fin 62 permet de réduire l'effort du servomoteur 6 lorsque celui-ci tend le ressort 51. Comme illustré à la figure 9a, les moyens de mise en tension peuvent aussi être commandés manuellement à l'aide du palonnier 60 agencé sur une surface d'un bord latéral 12. Il est ainsi possible pour le patient de débloquer le genou prothétique 1 en cas de défaillance du servomoteur 6.

Le genou prothétique 1 comporte avantageusement une jauge de contraintes agencée pour mesurer le couple exercé sur le tendeur rotatif 5. La jauge de contraintes est préférentiellement fixée au servomoteur 6.

Comme illustré à la figure 11, le genou prothétique 1 comporte avantageusement une carte électronique 61. La carte électronique 61 est préférentiellement disposée entre les deux bords latéraux 12. Le servomoteur 6 est avantageusement connecté électriquement à la carte électronique 61.

La partie proximale 10 présente un angle de pivotement entre la position d'extension et la position de flexion dans un plan sagittal du patient. Le genou prothétique 1 comporte avantageusement des moyens de mesure agencés pour mesurer l'angle de pivotement. Comme illustré à la figure 13, les moyens de mesure comportent de préférence un accéléromètre 7a et un gyromètre 7b montés sur la partie proximale 10. L'accéléromètre 7a et le gyromètre 7b sont connectés électriquement à la carte électronique 61, par exemple par une nappe électrique souple. Le servomoteur 6 peut ainsi moduler la tension mécanique exercée sur l'élément de liaison 4 par les moyens de mise en tension en fonction de l'angle de pivotement mesuré, et de la vitesse à laquelle il varie.
Selon une variante, les moyens de mesure peuvent comporter un capteur potentiométrique, de préférence de type bande résistive. Un avantage procuré par une bande résistive est d'éviter la présence d'une nappe électrique souple (connectant l'accéléromètre 7a et le gyromètre 7b à la carte électronique 61) au voisinage de la partie proximale 10. En effet, la nappe électrique est susceptible de se dégrader (voire se rompre) au contact de la partie proximale 10 après une multitude de flexions du genou prothétique 1. Le capteur potentiométrique peut être monté sur une pièce 3 proche de la partie proximale 10. A titre d'exemple, les moyens de mesure peuvent comporter un plot monté sur ressort, et relié à la partie proximale 10 de manière à glisser sur la bande résistive. La bande résistive peut être collée sur la pièce 3 proche de la partie proximale 10.

### Anticipation du début de la flexion du genou prothétique

Le genou prothétique 1 comporte avantageusement un capteur de force 8 agencé pour mesurer la force exercée par l'élément de liaison 4 sur la pièce 3 ou une pièce 3 parmi l'ensemble de pièces 3. Comme illustré à la figure 10, le capteur de force 8 est préférentiellement agencé sur un bord latéral 12 du genou prothétique 1. Le bord latéral 12 comporte préférentiellement des ouvertures 80 ménagées en son sein. Le capteur de force 8 comporte préférentiellement une pluralité de résistances électriques 81 fixées audit bord latéral 12, par exemple par collage, à l'intérieur des ouvertures 80. Les résistances électriques 81 sont préférentiellement câblées en pont de Wheatstone. Lorsque la pièce 3 présente la forme d'un tube, le capteur de force 8 peut être agencé à une extrémité du tube qui se tord lors de la flexion du genou prothétique 1.

Le genou prothétique 1 comporte avantageusement des moyens d'avertissement configurés pour avertir le patient dès lors que la force mesurée par le capteur de force 8 dépasse un seuil. Les moyens d'avertissement comportent de préférence un vibreur. Le vibreur est connecté électriquement à la carte électronique 61, par exemple par une nappe électrique souple. Le vibreur est préférentiellement ménagé au sein de la partie proximale 10 pour un meilleur ressenti du patient. Néanmoins, le vibreur peut également être monté sur un flanc 12 proche de la partie proximale 10 afin d'éviter une dégradation de la nappe électrique souple.

Le genou prothétique 1 peut également comporter un dispositif d'aide à la flexion configuré pour aider le patient âgé à s'asseoir. Ce dispositif peut par exemple être associé au capteur de force 8 pour assister le patient dès que la force mesurée dépasse un certain seuil. En alternative, le dispositif peut être directement commandé par le patient.

### Genou prothétique avec articulation polyaxiale

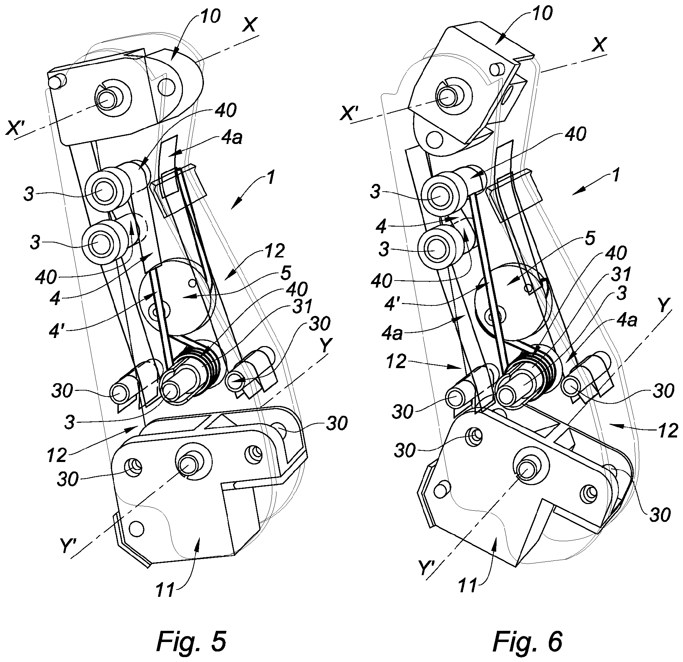

Le genou prothétique 1 comporte avantageusement :
- une partie distale 11, destinée à être reliée à un pied prothétique 20, la partie distale 11 étant mobile en rotation autour d'un second axe Y'-Y perpendiculaire à un plan sagittal du patient entre une position d'avancée et une position de recul du pied prothétique 20 ; la partie distale 11 étant mobile dans un premier sens de rotation de la position d'avancée vers la position de recul ;
- un élément de liaison additionnel 4a agencé pour relier la partie proximale 10 et la partie distale 11 de sorte que, lorsque la partie proximale 10 est mue dans le premier sens de rotation, la partie distale 11 est mue dans le premier sens de rotation.
Le second axe Y'-Y est parallèle au premier axe X'-X (les figures 5 et 6 sont trompeuses en raison du point de fuite).

La partie proximale 10 présente avantageusement une forme oblongue dans un plan sagittal du patient. En d'autres termes, la partie proximale 10 présente une portion plus longue que large dans un plan sagittal du patient. Pour rappel, un avantage procuré par la forme oblongue est d'obtenir un déplacement de la partie proximale 10 vers l'avant du patient avec :
- une amplitude importante dès le début de la flexion du genou prothétique 1 grâce à l'élément de liaison additionnel 4a se déplaçant en contact avec la portion large de la partie proximale 10, de dimension restreinte ;
- une amplitude plus faible jusqu'à la fin de la flexion grâce à l'élément de liaison additionnel 4a se déplaçant en contact avec la portion longue de la partie proximale 10, de dimension plus importante.
En outre, en fin de flexion, l'élément de liaison 4, 4' peut être fortement tendu, ce qui conduit à durcir l' « effet de frein ».
Ainsi, les dimensions de la portion longue et de la portion large de la partie proximale 10 peuvent être déterminées :
- selon l'amplitude souhaitée en début de flexion du déplacement de la partie proximale 10 vers l'avant du patient, et
- selon la tension souhaitée de l'élément de liaison 4 en fin de flexion.
La partie proximale 10 présente de préférence la forme d'un ellipsoïde ou d'un ovoïde.

Pour une sécurité supplémentaire pendant la marche au posé du talon (pour une version sans électronique par exemple), l'élément de liaison additionnel 4a et l'ensemble de pièces 3 sont avantageusement agencés de sorte que l'élément de liaison additionnel 4a forme des lacets autour de l'ensemble de pièces 3. L'élément de liaison additionnel 4a est avantageusement sélectionné dans le groupe comportant une sangle, une cordelette, une courroie. L'élément de liaison additionnel 4a est avantageusement résistant à la traction et à l'usure. Lorsque l'élément de liaison additionnel 4a est une sangle, celle-ci est par exemple réalisée en Cordura®.

La partie proximale 10 et la partie distale 11 présentent des vitesses de rotation dans le premier sens de rotation notées respectivement Vp₁ et Vd₁. Le genou prothétique 1 comporte avantageusement des organes 30 adaptées pour recevoir l'élément de liaison additionnel 4a, les organes 30 étant configurés de sorte que Vd₁ est strictement inférieure à Vp₁ avec de préférence *Vp*₁/*Vd*₁ ≥ 3. Les organes 30 forment avantageusement des poulies configurées en mouflage de manière à obtenir le rapport *Vp*₁/*Vd*₁ souhaité. Les organes 30 sont montés libres en rotation. Les organes 30 sont préférentiellement de forme tubulaire. Au moins un roulement 31 de roue libre peut être monté sur les organes 30 de manière à générer un effet de frein à la flexion du genou prothétique 1 via l'élément de liaison additionnel 4a.

L'invention ne se limite pas aux modes de réalisation exposés. L'homme du métier est mis à même de considérer leurs combinaisons techniquement opérantes, et de leur substituer des équivalents.

## Revendications

1. Genou prothétique (1) pour un patient amputé fémoral, comportant :
- une partie proximale (10) destinée à être reliée à une emboîture prothétique (2), la partie proximale (10) étant mobile en rotation autour d'un premier axe (X'-X) perpendiculaire à un plan sagittal du patient entre une position d'extension et une position de flexion du genou prothétique (1) ; la partie proximale (10) étant mobile dans un premier sens de rotation de la position d'extension vers la position de flexion, et mobile dans un second sens de rotation opposé de la position de flexion vers la position d'extension ;
- au moins une pièce (3), la pièce (3) étant fixe lorsque la partie proximale (10) est mue dans le premier sens de rotation ;
- un élément de liaison (4), agencé pour relier la partie proximale (10) et la pièce (3), l'élément de liaison (4) présentant une surface de contact (40) avec la pièce (3) ; l'élément de liaison (4) et la pièce (3) étant agencés de sorte que, lorsque la partie proximale (10) est mue dans le premier sens de rotation, l'élément de liaison (4) glisse sur la surface de contact (40) en subissant une première force de frottement,
**caractérisé en ce que** l'élément de liaison (4) et la pièce (3) sont en outre agencés de sorte que, lorsque la partie proximale (10) est mue dans le second sens de rotation, l'élément de liaison (4) glisse sur la surface de contact (40) en subissant une seconde force de frottement strictement inférieure à la première force de frottement.

2. Genou prothétique (1) selon la revendication 1, dans lequel les première et seconde forces de frottement, notées respectivement f₁ et f₂, présentent un ratio f₁/f₂ supérieur ou égal à 10.

3. Genou prothétique (1) selon l'une des revendications 1 ou 2, dans lequel la pièce (3) est mobile lorsque la partie proximale (10) est mue dans le second sens de rotation.

4. Genou prothétique (1) selon l'une des revendications 1 à 3, comportant des moyens de mise en tension agencés pour exercer une tension mécanique sur l'élément de liaison (4).

5. Genou prothétique (1) selon la revendication 4, comportant des moyens de commande configurés pour commander les moyens de mise en tension.

6. Genou prothétique (1) selon la revendication 5, dans lequel les moyens de mise en tension comportent un tendeur rotatif (5), et les moyens de commande comportent un servomoteur (6, 60) configuré pour modifier un couple exercé sur le tendeur rotatif (5).

7. Genou prothétique (1) selon la revendication 6, comportant une jauge de contraintes agencée pour mesurer le couple exercé sur le tendeur rotatif (5).

8. Genou prothétique (1) selon l'une des revendications 1 à 7, dans lequel la partie proximale (10) présente un angle de pivotement entre la position d'extension et la position de flexion dans un plan sagittal du patient ; le genou prothétique comportant des moyens de mesure agencés pour mesurer l'angle de pivotement, les moyens de mesure étant de préférence sélectionnés dans le groupe comportant un accéléromètre (7a), un gyromètre (7b), un capteur potentiométrique.

9. Genou prothétique (1) selon l'une des revendications 1 à 8, comportant un capteur de force (8, 81) agencé pour mesurer la force exercée par l'élément de liaison (4) sur la pièce (3).

10. Genou prothétique (1) selon la revendication 9, comportant des moyens d'avertissement configurés pour avertir le patient dès lors que la force mesurée par le capteur de force (8, 81) dépasse un seuil, les moyens d'avertissement comportant de préférence un vibreur.

11. Genou prothétique (1) selon l'une des revendications 1 à 10, comportant des moyens de rappel élastique agencés pour rappeler la partie proximale (10) dans la position d'extension.

12. Genou prothétique (1) selon l'une des revendications 1 à 11, dans lequel l'élément de liaison (4) est sélectionné dans le groupe comportant une sangle, une cordelette, une courroie.

13. Genou prothétique selon l'une des revendications 1 à 12, dans lequel l'élément de liaison (4) comporte une partie enroulée autour de la pièce (3), la partie enroulée étant de préférence une cordelette (4').

14. Genou prothétique (1) selon l'une des revendications 1 à 13, comportant un ensemble de pièces (3), les pièces (3) de l'ensemble étant fixes lorsque la partie proximale (10) est mue dans le premier sens de rotation ; l'élément de liaison (4) étant agencé pour relier la partie proximale (10) et l'ensemble de pièces (3), l'élément de liaison (4) présentant des surfaces de contact (40) avec l'ensemble de pièces (3) ; l'élément de liaison (4) et l'ensemble de pièces (3) étant agencés de sorte que, lorsque la partie proximale (10) est mue dans le premier sens de rotation, l'élément de liaison (4) glisse sur chaque surface de contact (40) en subissant la première force de frottement correspondante.

15. Genou prothétique (1) selon la revendication 14, dans lequel l'élément de liaison (4) et l'ensemble de pièces (3) sont agencés de sorte que l'élément de liaison (4) forme des lacets autour de l'ensemble de pièces (3).

16. Genou prothétique (1) selon l'une des revendications 1 à 15, comportant :
- une partie distale (11), destinée à être reliée à un pied prothétique (20), la partie distale (11) étant mobile en rotation autour d'un second axe (Y'-Y) perpendiculaire à un plan sagittal du patient entre une position d'avancée et une position de recul du pied prothétique (20) ; la partie distale (11) étant mobile dans un premier sens de rotation de la position d'avancée vers la position de recul ;
- un élément de liaison additionnel (4a) agencé pour relier la partie proximale (10) et la partie distale (11) de sorte que, lorsque la partie proximale (10) est mue dans le premier sens de rotation, la partie distale (11) est mue dans le premier sens de rotation.

17. Genou prothétique (1) selon la revendication 16 en combinaison avec la revendication 15, dans lequel l'élément de liaison additionnel (4a) et l'ensemble de pièces (3) sont agencés de sorte que l'élément de liaison additionnel (4a) forme des lacets autour de l'ensemble de pièces (3).

18. Genou prothétique (1) selon la revendication 16 ou 17, dans lequel l'élément de liaison additionnel (4a) est sélectionné dans le groupe comportant une sangle, une cordelette, une courroie.

19. Genou prothétique (1) selon l'une des revendications 16 à 18, dans lequel la partie proximale (10) et la partie distale (11) présentent des vitesses de rotation dans le premier sens de rotation notées respectivement Vp₁ et Vd₁ ; le genou prothétique (1) comportant des organes (30) adaptés pour recevoir l'élément de liaison additionnel (4a), les organes (30) étant configurés de sorte que Vd₁ est strictement inférieure à Vp₁ avec de préférence *Vp*₁/*Vd*₁ ≥ 3.

20. Genou prothétique (1) selon l'une des revendications 1 à 19, dans lequel la partie proximale (10) présente une forme oblongue dans un plan sagittal du patient.

## Patentansprüche

1. Knieprothese (1) für oberschenkelamputierte Patienten, die aufweist:
- einen proximalen Gelenkteil (10), der dazu vorgesehen ist, mit einem Prothesenschaft (2) verbunden zu sein, wobei der proximale Gelenkteil (10) um eine erste Achse (X' - X), die im rechten Winkel zu einer Sagittalebene des Patienten verläuft, zwischen einer Streckstellung und einer Beugestellung der Knieprothese (1) drehbeweglich ist; wobei der proximale Gelenkteil (10) in einer ersten Drehrichtung von der Streckstellung zu der Beugestellung hin beweglich ist und in einer zweiten, entgegengesetzten Drehrichtung von der Beugestellung zu der Streckstellung hin beweglich ist;
- mindestens ein Teil (3), wobei dieses Teil (3) feststehend ist, wenn der proximale Gelenkteil (10) in der ersten Drehrichtung bewegt wird;
- ein Verbindungselement (4), das dazu vorgesehen ist, den proximalen Gelenkteil (10) und das Teil (3) miteinander zu verbinden, wobei das Verbindungselement (4) eine Kontaktoberfläche (40) gegenüber dem Teil (3) aufweist; wobei das Verbindungselement (4) und das Teil (3) dergestalt vorgesehen sind, dass, wenn der proximale Gelenkteil (10) in der ersten Drehrichtung bewegt wird, das Verbindungselement (4) über die Kontaktoberfläche (40) gleitet und dabei einer ersten Reibungskraft unterliegt,
**dadurch gekennzeichnet,**
**dass** das Verbindungselement (4) und das Teil (3) ferner dergestalt vorgesehen sind, dass, wenn der proximale Gelenkteil (10) in der zweiten Drehrichtung bewegt wird, das Verbindungselement (4) über die Kontaktoberfläche (40) gleitet und dabei einer zweiten Reibungskraft unterliegt, die unbedingt geringer ist als die erste Reibungskraft.

2. Knieprothese (1) nach Anspruch 1, wobei die erste und die zweite Reibungskraft, die mit f₁ bzw. f₂ bezeichnet werden, in einem Verhältnis f₁/f₂ stehen, das größer oder gleich 10 ist.

3. Knieprothese (1) nach einem der Ansprüche 1 oder 2, wobei das Teil (3) beweglich ist, wenn der proximale Gelenkteil (10) in der zweiten Drehrichtung bewegt wird.

4. Knieprothese (1) nach einem der Ansprüche 1 bis 3, die Spannmittel aufweist, die dazu vorgesehen sind, das Verbindungselement (4) unter eine mechanische Spannung zu setzen.

5. Knieprothese (1) nach Anspruch 4, die Steuermittel aufweist, die dazu konfiguriert sind, die Spannmittel zu steuern.

6. Knieprothese (1) nach Anspruch 5, wobei die Spannmittel einen Drehspanner (5) umfassen und die Steuermittel einen Servomotor (6, 60) umfassen, der dazu vorgesehen ist, ein auf den Drehspanner (5) ausgeübtes Drehmoment zu modifizieren.

7. Knieprothese (1) nach Anspruch 6, die einen Spannungsmesser enthält, der vorgesehen ist, um das auf den Drehspanner (5) ausgeübte Drehmoment zu messen.

8. Knieprothese (1) nach einem der Ansprüche 1 bis 7, wobei der proximale Gelenkteil (10) einen Drehwinkel zwischen der Streckstellung und der Beugestellung in einer Sagittalebene des Patienten aufweist; wobei die Knieprothese Messmittel enthält, die dazu vorgesehen sind, den Drehwinkel zu messen, wobei die Messmittel vorzugsweise unter einem Beschleunigungsmesser (7a), einem Gyrometer (7b), einem potentiometrischen Sensor gewählt sind.

9. Knieprothese (1) nach einem der Ansprüche 1 bis 8, die einen Kraftsensor (8, 81) enthält, der dazu vorgesehen ist, die durch das Verbindungselement (4) auf das Teil (3) ausgeübte Kraft zu messen.

10. Knieprothese (1) nach Anspruch 9, die Meldemittel enthält, die dazu vorgesehen sind, den Patienten zu warnen, sobald die durch den Kraftsensor (8, 81) gemessene Kraft einen Schwellenwert überschreitet, wobei die Meldemittel vorzugsweise einen Vibrator enthalten.

11. Knieprothese (1) nach einem der Ansprüche 1 bis 10, die elastische Rückstellmittel enthält, die dazu vorgesehen sind, den proximalen Gelenkteil (10) in die Streckstellung zurück zu bringen.

12. Knieprothese (1) nach einem der Ansprüche 1 bis 11, bei der das Verbindungselement (4) unter einem Gurt, einer Schnur, einem Riemen gewählt ist.

13. Knieprothese (1) nach einem der Ansprüche 1 bis 12, bei der das Verbindungselement (4) einen um das Teil (3) aufgewickelten Abschnitt enthält, wobei der aufgewickelte Abschnitt vorzugsweise eine Schnur (4') ist.

14. Knieprothese (1) nach einem der Ansprüche 1 bis 13, die eine Gruppe von Teilen (3) enthält, wobei die Teile (3) der Gruppe fest stehen, wenn der proximale Gelenkteil (10) in der ersten Drehrichtung bewegt wird; wobei das Verbindungselement (4) dazu vorgesehen ist, den proximalen Gelenkteil (10) und die Gruppe von Teilen (3) miteinander zu verbinden, wobei das Verbindungselement (4) eine Kontaktoberfläche (40) gegenüber der Gruppe von Teilen (3) aufweist; wobei das Verbindungselement (4) und die Gruppe von Teilen (3) dergestalt vorgesehen sind, dass, wenn der proximale Gelenkteil (10) in der ersten Drehrichtung bewegt wird, das Verbindungselement (4) über jede Kontaktoberfläche (40) gleitet und dabei der ersten entsprechenden Reibungskraft unterliegt.

15. Knieprothese (1) nach Anspruch 14, wobei das Verbindungselement (4) und die Gruppe von Teilen (3) dergestalt ausgeführt sind, dass das Verbindungselement (4) Schlingen um die Gruppe von Teilen (3) bildet.

16. Knieprothese (1) nach einem der Ansprüche 1 bis 15, die aufweist:
- einen distalen Gelenkteil (11), der dazu vorgesehen ist, mit einem prothetischen Fuß (20) verbunden zu sein, wobei der distale Gelenkteil (11) um eine zweite Achse (Y' - Y), die im rechten Winkel zu einer Sagittalebene des Patienten verläuft, zwischen einer vorgeschobenen Stellung und einer rückwärtigen Stellung des prothetischen Fußes (20) drehbeweglich ist; wobei der distale Gelenkteil (11) in einer ersten Drehrichtung von der vorgeschobenen Stellung zu der rückwärtigen Stellung hin beweglich ist;
- ein zusätzliches Verbindungselement (4a), das dazu vorgesehen ist, den proximalen Gelenkteil (10) und den distalen Gelenkteil (11) dergestalt miteinander zu verbinden, dass, wenn der proximale Gelenkteil (10) in der ersten Drehrichtung bewegt wird, der distale Gelenkteil (11) in der ersten Drehrichtung bewegt wird.

17. Knieprothese (1) nach Anspruch 16 in Kombination mit Anspruch 15, bei der das zusätzliche Verbindungselement (4a) und die Gruppe von Teilen (3) dergestalt ausgeführt sind, dass das zusätzliche Verbindungselement (4a) Schlingen um die Gruppe von Teilen (3) bildet.

18. Knieprothese (1) nach Anspruch 16 oder 17, bei der das zusätzliche Verbindungselement (4a) unter einem Gurt, einer Schnur, einem Riemen gewählt ist.

19. Knieprothese (1) nach einem der Ansprüche 16 bis 18, bei der der proximale Gelenkteil (10) und der distale Gelenkteil (11) in der ersten Drehrichtung Drehgeschwindigkeiten aufweisen, die mit Vp₁ bzw. Vd₁ bezeichnet sind; wobei die Knieprothese (1) Organe (30) aufweist, die geeignet sind, das zusätzliche Verbindungselement (4a) aufzunehmen, wobei die Organe (30) dergestalt ausgeführt sind, dass Vd₁ unbedingt geringer ist als Vp₁, und vorzugsweise Vp₁ /Vd₁ ≥ 3.

20. Knieprothese (1) nach einem der Ansprüche 1 bis 19, bei der der proximale Gelenkteil (10) eine längliche Form in einer Sagittalebene des Patienten aufweist.

## Claims

1. Prosthetic knee joint (1) for an above-the-knee amputee patient, comprising:
- a proximal part (10) designed to be connected to a prosthetic socket (2), the proximal part (10) being mobile in rotation around a first axis (X'-X) perpendicular to a sagittal plane of the patient between an extended position and a flexion position of the prosthetic knee joint (1); the proximal part (10) being mobile in a first direction of rotation from the extended position to the flexion position, and mobile in an opposite second direction of rotation from the flexion position to the extended position;
- at least one part (3), the part (3) being arranged fixed when the proximal part (10) is moved in the first direction of rotation;
- a link part (4), arranged to connect the proximal part (10) and the part (3), the link part (4) presenting a contact surface (40) with the part (3);
the link part (4) and the part (3) being arranged so that when the proximal part (10) is moved in the first direction of rotation, the link part (4) slides on the contact surface (40) being subjected to a first friction force,
**characterized in that** the link part (4) and the part (3) further being arranged so that when the proximal part (10) is moved in the second direction of rotation, the link part (4) slides on the contact surface (40) being subjected to a second friction force strictly lower than the first friction force.

2. Prosthetic knee joint (1) according to claim 1, wherein the first and second friction forces, respectively noted f₁ and f₂, present a ratio f₁/f₂ higher than or equal to 10.

3. Prosthetic knee joint (1) according to the one of claims 1 or 2, wherein the part (3) is mobile when the proximal part (10) is moved in the second direction of rotation.

4. Prosthetic knee joint (1) according to one of claims 1 to 3, comprising tensioning means arranged to exert a mechanical tension on the link part (4).

5. Prosthetic knee joint (1) according to claim 4, comprising control means configured to control the tensioning means.

6. Prosthetic knee joint (1) according to claim 5, wherein the tensioning means comprise a rotary tensioner (5), and the control means comprise a servomotor (6, 60) configured to modify a torque exerted on the rotary tensioner (5).

7. Prosthetic knee joint (1) according to claim 6, comprising a stress gauge arranged to measure the torque exerted on the rotary tensioner (5).

8. Prosthetic knee joint (1) according to one of claims 1 to 7, wherein the proximal part (10) presents a pivoting angle between the extended position and the flexion position in a sagittal plane of the amputee patient; the prosthetic knee joint comprising measuring means arranged to measure the pivoting angle, the measuring means preferably being selected from the group comprising an accelerometer (7a), a gyrometer (7b), and a potentiometric sensor.

9. Prosthetic knee joint (1) according to one of claims 1 to 8, comprising the force sensor (8, 81) arranged to measure the force exerted by the link part (4) on the part (3).

10. Prosthetic knee joint (1) according to claim 9, comprising warning means configured to warn the amputee patient as soon as the force measured by the force sensor (8, 81) exceeds a threshold, the warning means preferably comprising a vibrator.

11. Prosthetic knee joint (1) according to one of claims 1 to 10, comprising flexible biasing means arranged to bias the proximal part (10) to the extended position.

12. Prosthetic knee joint (1) according to one of claims 1 to 11, wherein the link part (4) is selected from the group comprising a strap, a cord, or a belt.

13. Prosthetic knee joint according to one of claims 1 to 12, wherein the link part (4) comprises a part wound around the part (3), the wound part preferably being a cord (4').

14. Prosthetic knee joint (1) according to one of claims 1 to 13, comprising a set of parts (3), the parts (3) of the set being arranged fixed when the proximal part (10) is moved in the first direction of rotation; the link part (4) being arranged to connect the proximal part (10) and the set of parts (3), the link part (4) presenting contact surfaces (40) with the set of parts (3); the link part (4) and the set of parts (3) being arranged so that, when the proximal part (10) is moved in the first direction of rotation, the link part (4) slides on each contact surface (40) being subjected to the corresponding first friction force.

15. Prosthetic knee joint (1) according to claim 14, wherein the link part (4) and the set of parts (3) are arranged so that the link part (4) forms strings around the set of parts (3).

16. Prosthetic knee joint (1) according to one of claims 1 to 15, comprising:
- a distal part (11), designed to be connected to a prosthetic foot (20), the distal part (11) being mobile in rotation around a second axis (Y'-Y) perpendicular to a sagittal plane of the amputee patient between a forward movement position and a backward movement position of the prosthetic foot (20); the distal part (11) being mobile in a first direction of rotation from the forward movement position to the backward movement position;
- an additional link part (4a) arranged to connect the proximal part (10) and the distal part (11) so that, when the proximal part (10) is moved in the first direction of rotation, the distal part (11) is moved in the first direction of rotation.

17. Prosthetic knee joint (1) according to claim 16 in combination with claim 15, wherein the additional link part (4a) and the set of parts (3) are arranged so that the additional link part (4a) forms strings around the set of parts (3).

18. Prosthetic knee joint (1) according to claim 16 or 17, wherein the additional link part (4a) is selected from the group comprising a strap, a cord, or a belt.

19. Prosthetic knee joint (1) according to one of claims 16 to 18, wherein the proximal part (10) and the distal part (11) present speeds of rotation in the first direction of rotation respectively noted Vp₁ and Vd₁; the prosthetic knee joint (1) comprising means (30) designed to receive the additional link part (4a), the means (30) being configured so that Vd₁ is strictly lower than Vp₁ with preferably *Vp*₁/*Vd*₁ ≥ 3.

20. Prosthetic knee joint (1) according to one of claims 1 to 19, wherein the proximal part (10) presents an oblong shape in a sagittal plane of the amputee patient.
